# EUROPEAN PATENT SPECIFICATION

(11) **EP 4 265 286 B1**
(45) Date of publication and mention of the grant of the patent: **04.06.2025**
(21) Application number: 23169136.1
(22) Date of filing: 21.04.2023
(51) Int. Cl.: A61M 5/315, A61M 5/31

(54) **SAFETY APPARATUS FOR A MULTI-DOSE SYRINGE**
SICHERHEITSVORRICHTUNG FÜR EINE MEHRDOSISSPRITZE
DISPOSITIF DE SÉCURITÉ POUR UNE SERINGUE MULTIDOSE

(30) Priority: 21.04.2022 GB 202205849
(43) Date of publication of application: 25.10.2023
(73) Proprietor: MDS SYRINGES LTD, London N1 7GU (GB)
(72) Inventor: Moynihan, Laurence Redmond, HULL HU6 8QY (GB)
(74) Representative: Fry, David John

(56) References cited:
- AU-B2- 2014 328 035
- US-A- 4 475 905
- US-A1- 2020 306 453
- US-A1- 2021 146 058

## Description

This invention relates to safety apparatus for a multi-dose syringe.

Multi-dose syringes are known for use in administering a required substance, for example a drug, for a patient. There is a possibility that the intended doses may be exceeded both in terms of volume and also frequency of administration. This is especially so if the multi-dose syringe is being used in a pre-hospital environment. In addition, the contents of a loaded multi-dose syringe may not be adequately protected when the multi-dose syringe is in transit.

US4475905 describes an injection device that delivers a preselected dose from a conventional syringe. The device includes a sleeve having a first opening and a cavity sufficiently large to house a barrel of a conventional syringe. The sleeve further includes a second opening through which a cannula of the syringe extends and which is sufficiently small to retain the barrel of the syringe. A plunger-engaging cap is positioned within the sleeve in sliding relationship and engages a plunger of the syringe. The sleeve includes a slot extending longitudinally along the sleeve and the plunger-engaging cap includes a pin extending outwardly from the cap's surface and positioned within the slot. A stop is positionable at a predetermined location along the slot defining a preselected dosage such that when the plunger-engaging cap is pushed into the sleeve, the plunger of the syringe is pushed within the barrel of the syringe with the cap and plunger stopping when the pin of the cap engages the stop.

It is an aim of the present invention to reduce the above mentioned problems.
Accordingly, in one non-limiting embodiment of the present invention there is provided safety apparatus for a multi-dose syringe,
wherein the multi-dose syringe comprises:
   (i) a syringe barrel;
   (ii) a plunger which operates in the syringe barrel;
   (iii) a dose scale on the syringe barrel;
   (iv) screw threads on the syringe barrel; and
   (v) a dose dial which screws along the screw threads for dialling an injection dose on the dose scale;
wherein the safety apparatus comprises:
   (vi) a body for extending along the syringe barrel;
   (vii) a stopper member which is at a first end portion of the body and which is for stopping depression of the plunger in the syringe barrel; and
   (viii) a locating member which is at a second end portion of the body and which is for locating against the dose dial;
and wherein the safety apparatus is such that:
   (ix) the safety apparatus is securable in position on the multi-dose syringe by screwing the dose dial along the screw threads such that the dose dial tightens against the locating member and thereby causes the stopper member to engage the plunger such as to stop the depression of the plunger into the syringe barrel and thereby to stop injection of a dose from the multi-dose syringe;
characterised in that:
   (x) the locating member is a wedge formation for becoming wedged in position between the dose dial and the outside of the syringe barrel.

The safety apparatus of the present invention is advantageous in that it is able to be used on the multi-dose syringe such as to stop the depression of the plunger into the syringe barrel and thereby stop injection of a dose from the multi-dose syringe.

Thus the safety apparatus is able to provide safety in that it has to be removed from the multi-dose syringe before the multi-dose syringe can be operated. This helps to concentrate the mind of an operator of the multi-dose syringe and thereby helps to avoid incorrect dialling of a dose and also injection time periods which are too frequent. Also, when the safety apparatus is on the multi-dose syringe, accidental depression of the plunger into the syringe barrel is able to be avoided and thereby the contents of the multi-dose syringe are able to be protected against unwanted loss.

The safety apparatus may be one in which the body is an elongate body. Other shapes for the body may be employed. When the body is an elongate body, then the elongate body may be in the form of a column. Other shapes for the elongate body may be employed.

The safety apparatus may be one in which the stopper member engages the multi-dose syringe by extending underneath an operator squeeze formation on the plunger. The stopper member may be configured to otherwise engage the multi-dose syringe in order to stop the depression of the plunger into the syringe barrel.

In all embodiments of the invention, the stopper member may be a lug formation which extends from the body such as to engage the plunger. Other formations for the stopper member may be employed.

The safety apparatus may be one in which other types of locating member may be employed.

When the locating member is a wedge formation, then the wedge formation may comprise a face portion for engaging with the outside of the syringe barrel, and a curved portion for engaging with the dose dial. Other shapes for the wedge formation may be employed.

The safety apparatus may include a guide formation for guiding the safety apparatus during the positioning the safety apparatus on the syringe barrel.

The guide formation may comprise two guide arms which extend from the body at an angle to each other such as to extend around a part of the outside of the syringe barrel. Other configurations for the guide formation may be employed.

The present invention also extends to the combination of the safety apparatus of the present invention and the multi-dose syringe.

Embodiments of the invention will now be described solely by way of example and with reference to the accompanying drawings in which:
Figure 1 is an isometric view of safety apparatus of the present invention on a multi-dose syringe;

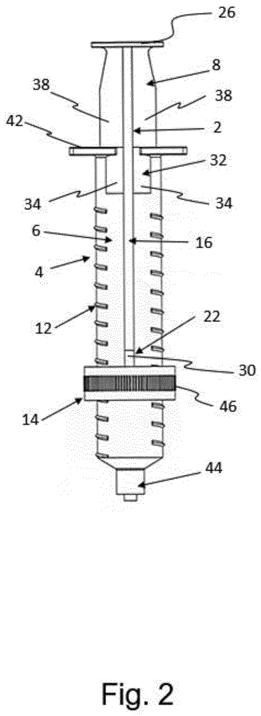

Figure 2 is a front view of the safety apparatus and the multi-dose syringe as shown in Figure 1;

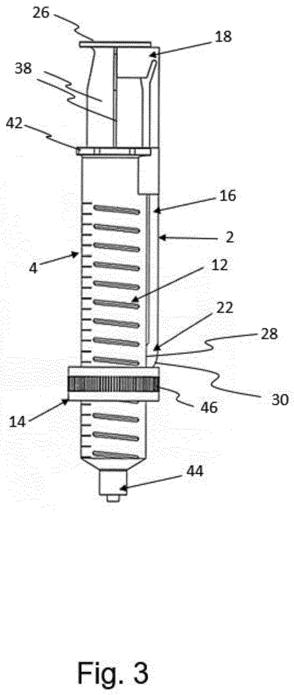

Figure 3 is a side view from the left of the safety apparatus and the multi dose syringe as shown in Figure 1;

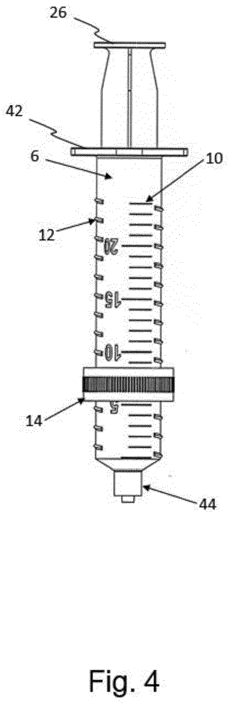

Figure 4 is a rear view of the safety apparatus and the multi-dose syringe as shown in Figure 1;
Figure 5 is a longitudinal section through the line A - A shown in Figure 1;

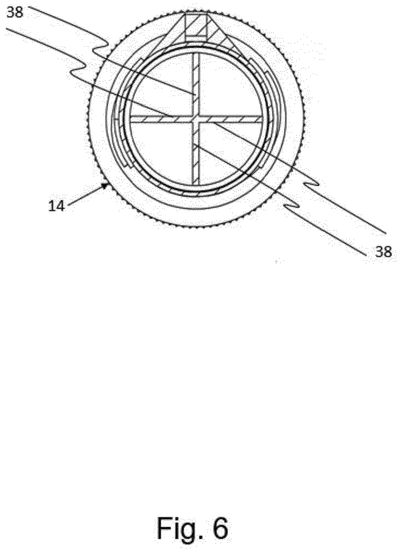

Figure 6 is a cross section through the line B - B shown in Figure 1; and

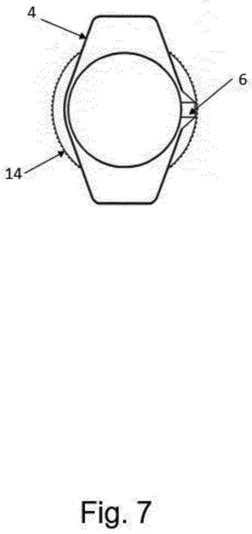

Figure 7 is a top view of the safety apparatus and the multi-dose syringe as shown in Figure 1.

Referring to the drawings, there is shown safety apparatus 2 for a multi-dose syringe 4.

The multi-dose syringe 4 is a known multi-dose syringe and it comprises a syringe barrel 6 and a plunger 8 which operates in the syringe barrel 6. The syringe barrel 6 has a dose scale 10 on the syringe barrel 6. Screw threads 12 are also on the syringe barrel 6. The multi-dose syringe 4 also comprises a dose dial 14 which screws along the screw threads 12 for dialling an injection dose on the dose scale 10.

The safety apparatus 2 comprises a body 16 for extending along the syringe barrel 6. The safety apparatus 2 also comprises a stopper member 18 which is at a first end portion 20 of the body 16. The stopper member 18 is for stopping depression of the plunger 8 into the syringe barrel 6.

The safety apparatus 2 further comprises a locating member 22 which is at a second end portion 24 of the body 16. The locating member 22 is for locating against the dose dial 14.

The safety apparatus 2 is such that it is securable in position on the multi-dose syringe 4 by screwing the dose dial 14 along the screw threads 12 such that the dose dial 14 tightens against the locating member 22. This thereby causes the stopper member 18 to engage the plunger 8 such as to stop the depression of the plunger 8 into the syringe barrel 6 and thereby to stop injection of a dose from the multi-dose syringe 4.

The body 16 is an elongate body. More specifically, the elongate body is a column.

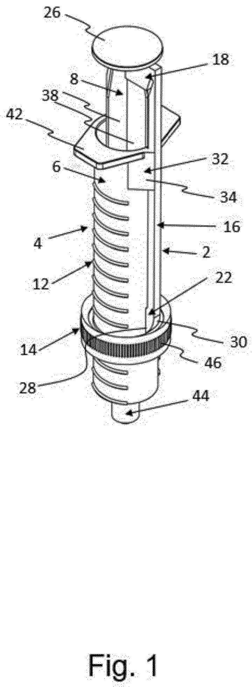

The stopper member 18 engages the multi-dose syringe 4 by extending underneath an operator squeeze formation 26 on the plunger 8. The operator squeeze formation 26 is in the form of a circular disc as can best be appreciated from Figure 1.

The stopper member 18 is a lug formation which extends from the body 16 such as to engage the plunger 8. More specifically, the stopper member 18 extends underneath the operator squeeze formation 26 on the plunger 8.

The locating member 22 is a wedge formation for becoming wedged in position between the dose dial 14 and the outside of the syringe barrel 6. The locating member 22 in the form of the wedge formation comprises a face portion 28 for engaging with the outside of the syringe barrel 6, and a curved portion 30 for engaging with the dose dial 14. As can best be seen from Figure 1, the curved portion 30 extends longitudinally along the second end portion 24 of the body 16 and curves towards the end of the body 16. The face portion 28 is a flat face portion and it is opposite the curved portion 30.

The safety apparatus 2 includes a guide formation 32 for guiding the safety apparatus 2 during the positioning of the safety apparatus 2 on the syringe barrel 6. The guide formation 32 comprises two guide arms 34, 36. The two guide arms 34, 36 extend from the body 16 at an angle to each other such as to extend around a part of the outside of the syringe barrel 6.

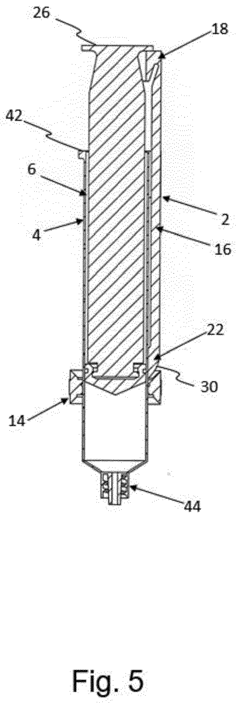

The multi-dose syringe 4 is such that the plunger 18 comprises vanes 38. The bottom of the plunger 8 is provided with a rubber seal 40 as shown in Figure 5. The rubber seal 40 provides sliding sealing contact along the inside of the syringe barrel 6 for injection purposes.

The syringe barrel 6 is provided with an operator squeeze formation 42 in the form of a flange. The two operator squeeze formations 26, 42 are squeezed together in order to cause depression of the plunger 8 along the syringe barrel 6 and thereby effect an injection of a dose of a liquid from the multi-dose syringe 4. The liquid in the multi-dose syringe 4 may be any suitable and appropriate required liquid including drugs such for example as morphine.

The multi-dose syringe 4 has an operating head 44 which is configured for receiving an injection needle (not shown) or a cannula.

The multi-dose syringe 4 may be used for any of the uses for known multi-dose syringes. Thus the multi-dose syringe may be used for injecting a wide variety of drugs for various ailments of human or animal patients.

It is to be appreciated that the embodiments of the invention described above with reference to the accompanying drawings have been given by way of example only and that modifications may be effected. Thus, for example, the body 16, the stopper member 18 and the locating member 22 may be of different constructional shapes to those shown. The dose dial 14 is shown with ribs 46 for facilitating rotation of the dose dial 14. Other gripping formations may be employed. Individual components shown in the drawings are not limited to use in their drawings and they may be used in other drawings and in all aspects of the invention. The invention also extends to the individual components mentioned and/or shown above, taken singly or in any combination.

## Claims

1. Safety apparatus for a multi-dose syringe,
wherein the multi-dose syringe (4) comprises:
(i) a syringe barrel (6);
(ii) a plunger (8) which operates in the syringe barrel (6);
(iii) a dose scale (10) on the syringe barrel (6);
(iv) screw threads (12) on the syringe barrel (6); and
(v) a dose dial (14) which screws along the screw threads (12) for dialling an injection dose on the dose scale (10);
wherein the safety apparatus (2) comprises:
(vi) a body (16) for extending along the syringe barrel (6);
(vii) a stopper member (18) which is at a first end portion (20) of the body (16) and which is for stopping depression of the plunger (8) in the syringe barrel (6); and
(viii) a locating member (22) which is at a second end portion (24) of the body (16) and which is for locating against the dose dial (14);
and wherein the safety apparatus is such that:
(ix) the safety apparatus (2) is securable in position on the multi-dose syringe (4) by screwing the dose dial (14) along the screw threads (12) such that the dose dial (14) tightens against the locating member (22) and thereby causes the stopper member (18) to engage the plunger (8) such as to stop the depression of the plunger (8) into the syringe barrel (6) and thereby to stop injection of a dose from the multi-dose syringe (4);
**characterised in that**:
(x) the locating member (22) is a wedge formation for becoming wedged in position between the dose dial and the outside of the syringe barrel.

2. Safety apparatus (2) according to claim 1 in which the body (16) is an elongate body.

3. Safety apparatus (2) according to claim 2 in which the elongate body (16) is a column.

4. Safety apparatus (2) according to any one of the preceding claims in which the stopper member (18) engages the multi-dose syringe (4) by extending underneath an operator squeeze formation (26) on the plunger (8).

5. Safety apparatus (2) according to any one of the preceding claims in which the stopper member (18) is a lug formation which extends from the body (16) such as to engage the plunger (8).

6. Safety apparatus (2) according to any one of the preceding claims in which the wedge formation (22) comprises a face portion (28) for engaging with the outside of the syringe barrel (6), and a curved portion (30) for engaging with the dose dial (14).

7. Safety apparatus (2) according to any one of the preceding claims and including a guide formation (32) for guiding the safety apparatus (2) during the positioning of the safety apparatus on the syringe barrel (6).

8. Safety apparatus (2) according to claim 7 in which the guide formation (32) comprises two guide arms (34, 36) which extend from the body (16) at an angle to each other such as to extend around a part of the outside of the syringe barrel (6).

9. The combination of safety apparatus (2) according to any one of the preceding claims and the multi-dose syringe (4).

## Patentansprüche

1. Sicherheitsvorrichtung für eine Mehrdosisspritze,
wobei die Mehrdosis-Spritze (4) Folgendes umfasst:
(i) einen Spritzenzylinder (6);
(ii) einen Kolben (8), der im Spritzenzylinder (6) eingesetzt wird;
(iii) eine Dosierskala (10) am Spritzenzylinder (6);
(iv) Schraubgewinde (12) am Spritzenzylinder (6); und
(v) eine Dosier-Drehscheibe (14), die entlang der Schraubengewinde (12) geschraubt wird, um eine Injektionsdosis auf der Dosierskala (10) einzustellen;
wobei die Sicherheitsvorrichtung (2) Folgendes umfasst:
(vi) einen Körper (16), der sich entlang des Spritzenzylinders (6) erstreckt;
(vii) ein Stoppelement (18), das sich an einem ersten Endabschnitt (20) des Körpers (16) befindet und zum Stoppen des Niederdrückens des Kolbens (8) in dem Spritzenzylinder (6) dient; und
(viii) ein Positionierungselement (22), das sich an einem zweiten Endabschnitt (24) des Körpers (16) befindet und zum Positionieren gegen die Dosis-Drehscheibe (14) dient;
und wobei die Sicherheitsvorrichtung so beschaffen ist, dass:
(ix) die Sicherheitsvorrichtung (2) an der Mehrdosis-Spritze (4) durch Schrauben der Dosis-Drehscheibe (14) entlang der Schraubgewinde (12) in ihrer Position gesichert werden kann, so dass sich die Dosis-Drehscheibe (14) gegen das Positionierungselement (22) festzieht und dadurch das Stoppelement (18) veranlasst, mit dem Kolben (8) in Eingriff zu kommen, um das Niederdrücken des Kolbens (8) in den Spritzenzylinder (6) zu stoppen und dadurch die Injektion einer Dosis aus der Mehrdosis-Spritze (4) zu stoppen;
**dadurch gekennzeichnet, dass**:
(x) das Positionierungselement (22) in einer Keilformation ist, um sich zwischen der Dosis-Drehscheibe und der Außenseite des Spritzenzylinders zu verkeilen.

2. Sicherheitsvorrichtung (2) nach Anspruch 1, bei der der Körper (16) ein länglicher Körper ist.

3. Sicherheitsvorrichtung (2) nach Anspruch 2, bei der der längliche Körper (16) eine Säule ist.

4. Sicherheitsvorrichtung (2) nach einem der vorhergehenden Ansprüche, bei der das Stoppelement (18) mit der Mehrdosis-Spritze (4) in Eingriff kommt, indem es sich unter eine Druckformation (26) für den Bediener auf dem Kolben (8) erstreckt.

5. Sicherheitsvorrichtung (2) nach einem der vorhergehenden Ansprüche, bei der das Stoppelement (18) eine Ansatzformation ist, die sich von dem Körper (16) erstreckt, um mit dem Kolben (8) in Eingriff zu kommen.

6. Sicherheitsvorrichtung (2) nach einem der vorhergehenden Ansprüche, bei der die Keilformation (22) einen Flächenabschnitt (28) zum Eingriff mit der Außenseite des Spritzenzylinders (6) und einen gekrümmten Abschnitt (30) zum Eingriff mit der Dosier-Drehscheibe (14) umfasst.

7. Sicherheitsvorrichtung (2) nach einem der vorhergehenden Ansprüche und mit einer Führungsformation (32) zum Führen der Sicherheitsvorrichtung (2) bei der Positionierung der Sicherheitsvorrichtung am Spritzenzylinder (6).

8. Sicherheitsvorrichtung (2) nach Anspruch 7, bei der die Führungsformation (32) zwei Führungsarme (34, 36) umfasst, die sich von dem Körper (16) in einem Winkel zueinander erstrecken, so dass sie sich um einen Teil der Außenseite des Spritzenzylinders (6) erstrecken.

9. Kombination aus der Sicherheitsvorrichtung (2) nach einem der vorhergehenden Ansprüche und der Mehrdosis-Spritze (4).

## Revendications

1. Un appareil de sécurité pour une seringue multi-dose,
dans lequel la seringue multi-dose (4) comprend :
(i) un corps de seringue (6) ;
(ii) un piston (8) qui fonctionne dans le corps de la seringue (6) ;
(iii) une échelle graduée (10) sur le corps de la seringue (6) ;
(iv) des filetages (12) sur le corps de la seringue (6) ;
(v) un cadran de dosage (14) qui se visse le long des filetages (12) pour définir une dose d'injection sur l'échelle graduée (10) ;
dans lequel l'appareil de sécurité (2) comprend :
(vi) un corps (16) destiné à s'étendre dans le corps de la seringue (6) ;
(vii) un élément de butée (18) situé à une première extrémité (20) du corps (16) et servant à empêcher d'appuyer sur le piston (8) dans le corps de la seringue (6) ;
(viii) un élément de localisation (22) situé à une seconde extrémité (24) du corps (16) et servant à se positionner contre le cadran de dosage (14) ;
et dans lequel l'appareil de sécurité est conçu de manière à ce que :
(ix) l'appareil de sécurité (2) peut être fixé en position sur la seringue multi-dose (4) en vissant le cadran de dosage (14) le long des filetages (12) de façon à serrer le cadran de dosage (14) contre l'élément de localisation (22), ce qui entraîne la venue en prise de l'élément de butée (18) contre le piston (8), de manière à empêcher d'appuyer sur le piston (8) dans le corps de la seringue (6), et donc à arrêter l'injection d'une dose à partir de la seringue multi-dose (4) ;
**caractérisé en ce que** :
(x) l'élément de localisation (22) est une formation en coin destinée à se loger en position entre le cadran de dosage et l'extérieur du corps de la seringue.

2. Appareil de sécurité (2) selon la revendication 1, dans lequel le corps (16) est un corps allongé.

3. Appareil de sécurité (2) selon la revendication 2, dans lequel le corps allongé (16) est une colonne.

4. Appareil de sécurité (2) selon l'une des revendications précédentes, dans lequel l'élément de butée (18) est en prise avec la seringue multi-dose (4) en s'étendant sous la zone de pression exercée par l'opérateur (26) sur le piston (8).

5. Appareil de sécurité (2) selon l'une des revendications précédentes, dans lequel l'élément de butée (18) prend la forme d'un ergot s'étendant depuis le corps (16) pour venir en prise avec le piston (8).

6. Appareil de sécurité (2) selon l'une des revendications précédentes, dans lequel la formation en coin (22) comporte une face (28) destinée à venir en prise avec l'extérieur du corps de seringue (6) et une partie incurvée (30) destinée à venir en prise avec le cadran de dosage (14).

7. Appareil de sécurité (2) selon l'une des revendications précédentes, comprenant un élément de guidage (32) destiné à guider l'appareil de sécurité (2) lors de son positionnement sur le corps de seringue (6).

8. Appareil de sécurité (2) selon la revendication 7, dans lequel l'élément de guidage (32) comprend deux bras de guidage (34, 36) qui s'étendent pour former un angle l'un par rapport à l'autre depuis le corps (16), de façon à entourer partiellement l'extérieur du corps de la seringue (6).

9. L'association de l'appareil de sécurité (2) selon l'une des revendications précédentes et de la seringue multi-dose (4).
